(19) Europäisches Patentamt
European Patent Office
Office européen des brevets

(11) **EP 4 171 387 B1**

(12) **EUROPEAN PATENT SPECIFICATION**

(45) Date of publication and mention
of the grant of the patent:
**07.08.2024  Bulletin 2024/32**

(21) Application number: **21732083.7**

(22) Date of filing: **18.06.2021**

(51) International Patent Classification (IPC):
**A61B 8/00** *(2006.01)*      **A61B 8/08** *(2006.01)*

(52) Cooperative Patent Classification (CPC):
**A61B 8/4483; A61B 8/469; A61B 8/52; A61B 8/56;**
A61B 8/0883; A61B 8/54

(86) International application number:
**PCT/EP2021/066538**

(87) International publication number:
**WO 2022/002627 (06.01.2022 Gazette 2022/01)**

(54) **METHOD AND SYSTEM FOR DATA TRANSFER REDUCTION IN ULTRASOUND IMAGING**

VERFAHREN UND SYSTEM ZUR DATENÜBERTRAGUNGSREDUKTION BEI DER
ULTRASCHALLBILDGEBUNG

PROCÉDÉ ET SYSTÈME DE RÉDUCTION DE TRANSFERT DE DONNÉES DANS L'IMAGERIE À
ULTRASONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.06.2020  EP 20182794**

(43) Date of publication of application:
**03.05.2023  Bulletin 2023/18**

(73) Proprietor: **Koninklijke Philips N.V.**
**5656 AG Eindhoven (NL)**

(72) Inventors:
• **VLUTTERS, Ruud**
**5656 AE Eindhoven (NL)**

• **BERA, Deep**
**5656 AE Eindhoven (NL)**
• **VAN RENS, Antonia, Cornelia**
**5656 AE Eindhoven (NL)**

(74) Representative: **Philips Intellectual Property &
Standards**
**High Tech Campus 52**
**5656 AG Eindhoven (NL)**

(56) References cited:
**US-A- 5 873 830    US-A- 6 123 670**

**Description**

FIELD OF THE INVENTION

The invention relates to a method for data transfer reduction in an ultrasound (US) imaging system and a corresponding system.

BACKGROUND OF THE INVENTION

In conventional US imaging, a 2D image is obtained by performing about 200 pulse-echo measurements by sending transmit pulses into 200 different directions within a plane covering a 90° angle. Thus, a single line is acquired for each ultrasound transmit event (single line acquisition, SLA). In this way, a 2D image with an imaging depth of 130 mm can be obtained at a frame rate of approximately 30 Hz. In ultrasound imaging of the heart, such a frame rate is sufficient to visualize the overall motion of the heart. However, some cardiac phases are very short lived and are known to be associated with very fast motion and deformation of the cardiac muscle and valves. An accurate study of these cardiac phases requires a higher frame rate.

Therefore, ultrafast ultrasound imaging techniques have been developed, such as parallel beamforming (i.e., multiple line acquisition, MLA). In this approach, multiple image lines are reconstructed simultaneously for each transmit pulse. To accommodate the increased number of parallel receive beams, the transmit beam must be broadened (e.g. by reducing the transmit aperture). However, the maximal number of parallel receive beams remains limited (e.g. 2, 4, or 8) because of the smaller aperture transmitting less energy. As an alternative, plane-wave or diverging-wave imaging has been proposed. Although this technique allows for a high degree of parallel receive beamforming, the spatial resolution of the image degrades significantly because of the very broad transmit beam. To compensate for this effect, different transmit beams are typically compounded, resulting in an overall gain in frame rate.

Generally, ultrafast imaging requires a relatively high number of transmissions (more than 10 for 2D imaging, more than 100 for 3D imaging) to produce images with acceptable quality. Normally, ultrafast imaging techniques are operated at a frame rate greater than 1000 Hz. Therefore, the number of insonifications per sec is greater than $10^4$ for 2D and greater than $10^5$ for 3D. In order to achieve sufficient penetration depth and in order to do harmonic imaging at depth, the used surface pressures have to be high. For example, it may take about 1.5W/mm$^2$ to generate a surface pressure of 1.5 MPa with a transducer aperture of 200mm$^2$ (assuming a power efficiency of the high-voltage driver of 30% and an on-off duty cycle of the pulser of 0.2%). Hence, a significant amount of acoustic energy is needed to insonify the field-of-view. When operating at a high frame rate, the acoustic power has to be generated many times.

These requirements lead to a higher power dissipation in the ultrasound (US) probe. Particularly in miniaturized, handheld and/or wearable (wireless) imaging systems, it is important to limit probe costs and power dissipation. A typical USB handheld probe limits the power dissipation to 2-3 W in order to be able to provide the power via the USB3 interface. Without active cooling, a limitation of 2-3 W is also needed in order to comply with medical regulations related to (short-term) skin heating. A wireless patch probe for monitoring applications may even limit the power dissipation to 100 mW in order to safeguard battery lifetime and prevent long-term skin heating.

Furthermore, the ultrafast ultrasound techniques result in an increased data-transfer burden, which standard technology may not yet support. Especially for matrix transducers that consist of more than 1000 transducer elements, the potential amount of radiofrequency (RF) data is extremely high. To achieve a high spatial resolution, the raw data of many transducer elements (channels) needs to be collected and processed independently. This will lead to high dissipation and potentially complex hardware, e.g. multiple Gbps serial data links.

It would therefore be desirable to limit the number of insonifications, as well as the amount of RF data that is generated, processed and transferred. Doing so, also reduces probe costs as simpler hardware components can be used. As the dominant power consumption is due to the insonification, it also is important to collect all relevant echo information to avoid the need for additional insonifications.

In the prior art, region-of-interest based image compression has been proposed (e.g. Heng-Ming Tails, Men Long, Wei He, and Hao Yarg, "AN EFFICIENT REGION OF INTEREST CODING FOR MEDICAL IMAGE COMPRESSION", Proceedings of the Second Joint EMEISEIMES Conference Houston, TX, USA * October 2+26,2W2; and "Review on Region of Interest Coding Techniques for Medical Image Compression", International Journal of Computer Applications (0975 - 8887) Volume 134 - No. 10, January 2016). In this approach, a region of an image (the region of interest, ROI) gets encoded with higher quality (possibly lossless) than the non-ROI region (typically compressed with a lossy method). This is usually achieved by allocating more bits to the ROI compared to the non-ROI region.

US 2016/0262720 A1 concerns ultrasound imaging and proposes to separately scan an entire volume and a sub-volume, wherein the sub-volume is scanned with different settings for beamforming parameters, thereby allowing for greater image quality for the sub-volume while providing context from the entire volume. Volume and sub-volume slices may than be interleaved.

US 5 873 830 A discloses an ultrasound system and method, wherein a region of interest is selected. A first set of imaging parameters is applied inside the region of interest to improve spatial resolution, while a different set of parameters is applied outside of the region of interest. A composite image, comprising the region of interest and the region outside the region of interest is then assembled.

However, even with such advanced methods, the amount of data that needs to be processed and transferred may still be a limiting factor for applicability and the goal to minimize associated costs.

OBJECT OF THE INVENTION

It is, therefore, an object of the invention to provide a method and related system for ultrasound imaging that reduces the power consumption of a US probe and the amount of data that needs to be transferred, while at the same time providing high-quality US images with sufficient temporal and spatial resolution as well as coverage of sufficiently large fields of view.

SUMMARY OF THE INVENTION

This object is met or exceeded by a method for US imaging according to claim 1 and a US imaging system according to claim 15. Any features, advantages or alternative embodiments described herein in relation to the claimed method are also applicable to the system and vice versa.

According to the invention, a method is provided for ultrasound imaging using an ultrasound system comprising a US probe having a transducer array and an analogue-to-digital converter (ADC) array, wherein the US probe is configured to insonify an imaging region, receive echo signals, digitize the received echo signals with the ADC array and transfer the digitized radiofrequency (RF) data to a data processing unit via a bandwidth-limited channel, wherein the data processing unit is adapted to beamform the RF data, the method comprising the following steps:

- acquiring at least one scouting image of the imaging region, the scouting image including an anatomical structure of interest;
- selecting at least one region of interest (ROI) within the scouting image, wherein the at least one ROI includes a portion of the anatomical structure;
- setting a transmit (TX) scheme and a receive (RX) scheme for US imaging of the imaging region, wherein the at least one ROI is insonified with different TX and RX settings than the imaging region outside the at least one ROI, and
- acquiring a plurality of US images according to the TX scheme and the RX scheme.

The US probe is configured to transfer to the data processing unit only the received RF data corresponding to a depth range of the at least one ROI for at least some of the stipulated receive beams reflected from the ROI, and is configured to transfer the received RF data corresponding to the full depth of the imaging region for at least some of the receive beams reflected from the imaging region outside the at least one ROI.

The above steps are usually executed in the order in which they are given, except that the TX scheme and the RX scheme may be configured in any order.

An important application of the invention is echocardiography. Considering the heart as a group of fast-moving objects, the valves of the heart move faster than the atrial/ventricular walls. Hence, in this and other applications, not all the parts of a moving anatomical structure need a fast or ultrafast imaging technique. Therefore, the invention provides a method, in which, first, one or several ultrasound images, referred to as scouting image(s), is/are acquired of an anatomical structure of interest, for example the entire heart. On this US image, a region of interest is selected which includes a portion of the anatomical structure, preferably a portion of particular interest, or a portion which moves faster than the rest of the anatomical structure. The ROI usually covers a part of the imaging region of the scouting image(s), for example 5% to 40% thereof. Preferably, the ROI may include a part of the anatomical structure that needs to be imaged with different scan settings, in particular different transmit and/or receive settings (also referred to as transmit and receive parameters) in order to obtain images with higher temporal resolution and/or better-quality from the ROI than for the rest of the imaging region. The TX and RX scheme comprise for example such settings as the general imaging/measurement mode (e.g. plane wave, diverging wave, Dopper imaging), the number, type and width of the TX and RX beams, frame rate/temporal resolution, transmit power, pulse repetition rate, focusing scheme etc.

The ROI may be selected manually or automatically. There may also be more than one ROI, e.g. 2 to 5 ROI's. Based on the location of the one or several ROIs within the imaging region in relation to the US probe, and the desired image quality or temporal resolution for the ROI, a transmit and receive scheme for US imaging of the imaging region is then set, preferably automatically according to pre-defined user specifications, wherein the transmit and receive scheme leads to a scanning of the ROI with different TX and RX settings, in particular with greater temporal resolution, contrast, power, focusing scheme or with generally different types of transmit beams than the imaging region outside the ROI. For example, based on the spatio-temporal location of the ROI, the transmit beams illuminating the ROI may be scheduled at smaller time intervals, so that RF data is acquired at a faster rate from the ROI. For the other parts of the anatomical structure, the stipulated transmit beams are scheduled at larger time intervals to match the slower speed of their movement.

Moreover, the receive scheme comprises configuring the US probe to transfer to the data processing unit only the received RF data corresponding to a depth range of the ROI for at least some of the stipulated receive beams reflected from the ROI, i.e. for the receive beams used to reconstruct images of the ROI. The depth direction corresponds to the distance from the US probe. By contrast, for non-ROI regions, at least some, preferably all, receive beams are digitized and transferred to the data processing unit (DPU). In other words, the receive scheme can be adjusted to only keep samples in the depth range corresponding to the ROI of the stipulated transmit beams. Thereby, the data transfer rate to the DPU is significantly reduced. Once digitized, the RF data may be shifted in time and interleaved as necessary for reconstructing a video stream of US images.

Accordingly, the invention describes a method that may reduce the number of transmit (TX) events, preferably resulting in faster frame rates, and reduces the amount of RF data which needs to be transferred from the probe to the data processing unit, thereby also reducing the amount of beamforming calculations that need to be performed. This is achieved by an adaptive TX-RX scheme, which achieves an improvement in data compression and power consumption by: 1) An optimized number of transmissions or TX/RX settings, and 2) the reconstruction of a limited depth range, resulting in a reduction in the RF data transfer load.

To realize these advantages, the invention is carried out with a digital US probe, i.e. a US probe with in-probe digitization of the received echo signals (RF signals). The digitized RF data is transferred to a data processing unit. In a next step, the digitized RF data may be processed and blended together into a continuous video stream showing the ROI region with better image quality, for example with a higher temporal resolution, than the non-ROI regions, i.e. the imaging region outside the ROI. In an embodiment, the data processing unit is adapted to beamform the RF data into a plurality of US images. In a next step, the plurality of US images are blended together into the video stream. In preferred embodiments, the data processing unit is not part of the ultrasound probe, but is situated within the main ultrasound system and is connected to the probe via a digital interface. Such digital interface may comprise a data connection or channel having limited bandwidth, i.e. a maximum data-rate, such as a serial data link having a defined maximum number of Gbps. In other words, the US probe and the data processing unit (DPU) are preferably accommodated in separate housings connected by a data cable or even by a wireless data connection. Accordingly, the method of the invention can be realised by a simplified digital front end, e.g. a digital US probe.

The scouting image(s) can be acquired with any imaging scheme, preferably in B-mode, and may have a lower resolution or lower frame rates in order to limit the data rates involved. However, the scouting image(s) of the imaging region (including the anatomical structure) may also be one or several of the plurality of US images obtained by carrying out the method of the invention, and therein the position of the ROI may be again detected and, if necessary, adjusted.

In an embodiment, several scouting images are acquired, so that a fast-moving region or portion of an anatomical structure can be detected, e.g. automatically by a computer vision or artificial intelligence (AI) algorithm, and selected as the region-of-interest (ROI). Such algorithms may be adapted specifically for a specific clinical application. Alternatively, the ROI may be manually selected, for example by a user displaying the scouting image (s) on a screen and setting one or several landmarks on the portion of interest. The user may also outline the ROI by dragging a graphical object such as a circle or square over the ROI, or drawing the boundaries of the ROI with the cursor.

Next, given the ROI, different transmit and receive settings are set for the ROI and the imaging region outside the ROI (non-ROI region).
s In other words, the transmit scheme and receive scheme according to an embodiment of the inventive method comprises two types of insonifications: type A for the complete imaging region, and type B for the ROI.

Type A: In a first series of insonifications, a target anatomical structure (e.g. a heart) is insonified, for example using identical plane wave transmits. The RF data from all transducers (e.g. 128 channels) may be used for the complete imaging region. The digitization and beamforming may be performed at a moderate resolution in order to limit the amount of data collected. By comparing and interpreting the data of sequential insonifications or scouting images, the system (in particular a controller of the US system) is able to detect the fast-moving areas, e.g. heart valves.

Type B: In a second series of insonifications, for example a focused transmit beam may be transmitted, and data from a limited number of RF signal channels may be collected. The data may be digitized and transferred at a relatively high resolution. The data acquisition and transfer only happens for a limited time period, corresponding to a limited depth range, to address a particular region of interest. For example, acquisition may start 50 $\mu$s after the insonification event and may last for 20 $\mu$s (real time).

It may take 1000 $\mu$s to transfer the data of insonification type A, and 100 $\mu$s to transfer the data of insonification type B. The controller is aware of the in-probe data storage capacity (if any) and the available transmission rates between the probe and the DPU and may adapt the timing for new insonification requests accordingly. Plane wave insonifications (e.g. for type A) and targeted insonifications (type B) may be generated in a regular time-interleaved manner. However, the interleaving may also be irregular/dynamic, and/or may be triggered by the momentary signal characteristics. The momentary signal characteristics may in particular be the speed of movement detected within a particular region of the imaging region. Artificial intelligence may be used to recognize these momentary signal characteristics to support the intelligent image formation or data detection.

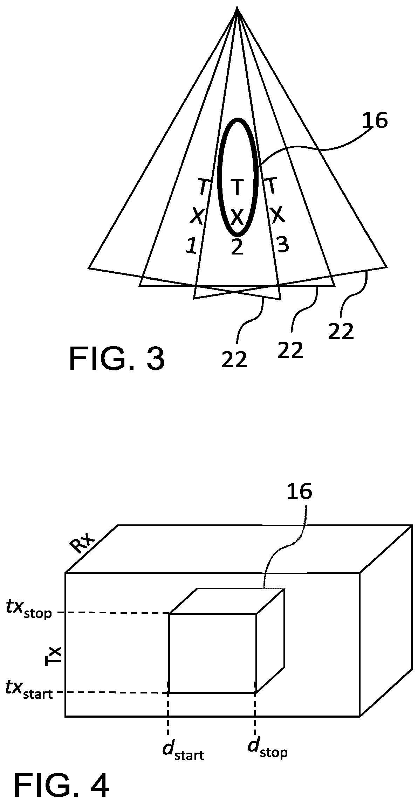

The amount of the data reduction is illustrated in Fig. 4: In the cuboid shown therein, the fast-time axis or depth axis is shown horizontally, the vertical axis shows the transmit events, and the third axis is the receive element axis. The full cuboid shows the full RF data array that would be acquired for a 2D image of the imaging region, in the case of multi-angled plane wave or diverging wave imaging. The smaller cube corresponds to the RF data transferred for at least some of the ROI scans. It is evident that the smaller cube is reduced in the depth direction, and on the transmit axis, because, as the ROI will typically not cover the complete azimuth, the transmit scheme will often require fewer transmit events to image the ROI than the complete imaging region.

In some embodiments, the cube representing the RF data to be transferred for the ROI may also be reduced in the receive element axis. In other words, when receiving RF data from a transmit beam illuminating the ROI, only a subset of the receive elements may be active, and/or only the data from a limited number of signal channels is collected and digitized. In other words, only the RF data from a part of the transducer elements is transferred to the DPU for at least some of the TX/RX events which illuminate the ROI. Thereby, the data transfer load can be further reduced.

By only transferring the RF data corresponding to a certain depth range (d, also called fast-time) and only when transmitting into certain transmit angles, one can reduce the amount of data to be transferred by:

$$R_{frame} = \left( \frac{d_{stop} - d_{start}}{d_{full}} \right) \left( \frac{tx_{stop} - tx_{start}}{tx_{full}} \right)$$

Thereby, the data transfer rate can be significantly reduced. For example, in a US probe with 128 transducer elements, a sound frequency of 8 GHz, sampled at 4 samples per wave with 12-bit resolution, the resulting data rate would be 8Mx128x4x12 bits/sec = 49 Gbit/sec. By reducing the amount of data transferred for those scans concerning the ROI, the data rate though the cable of the interface between probe and DPU can be reduced to e.g. less than 20 Gbit/sec, more preferred less than 10 Gbit/sec.

After digitization and transfer to the DPU, the digitized RF data will be beamformed into a plurality of US images. Since the data from the ROI region comprises less data, the beamforming calculations will also be computationally less expensive. One can apply beamforming on the RF data from the ROI by setting the data corresponding to the depth range outside the ROI to 0. Thereby, one receives an image of the imaging region, with the non-ROI region in black. This type of image may straightforwardly be blended with a beamformed US image of the entire imaging region. Alternatively, one can also beamform only the ROI region, resulting in a smaller image. This results in less beamforming calculations, but makes the video blending step more complex, in particular with respect to blending at the right location in the full image.

The result of the method is a plurality, in particular a time sequence, of US images showing the entire imaging region, but the ROI in better quality, in a different imaging or measuring mode, and/or at higher temporal resolution. The higher temporal resolution for the non-ROI region is achieved by upsampling, as described in more detail below, and therefore does not contain more information. The ROI region, however, has been sampled at a higher temporal resolution in some embodiments, and therefore, the fast-moving portion of the anatomical structure can be analysed. Accordingly, ultrafast US imaging can be realized at a lower required data transfer rate, resulting in less power dissipation, but without compromising on frame rate.

According to an embodiment, the TX scheme is configured such that the at least one ROI is insonified with a higher temporal resolution and/or with different types of transmit beams and/or with a different pulse-repetition rate than the imaging region outside the at least one ROI. In a preferred embodiment, the transmit parameters are configured such that the ROI is insonified more often than the non-ROI regions. According to the invention, a frame rate of about 60-1000 Hz, preferably 120-500 Hz can be realized in the ROI, which corresponding to ultrafast imaging, but without the higher power consumption and data transfer rate that would be required if the complete imaging region were imaged with such a high frame rate. In addition, or alternative thereto, depending on the location of the ROI with regard to the US probe, the transmit power and/or the transmit focusing scheme may be adapted. For example, focused TX may help to do harmonic imaging in the ROI. The pulse-repetition rate is the number of ultrasound pulses emitted per second. The pulse repetition rate is limited by the speed of sound in the medium, e.g. the tissue of a human body or an anatomical structure, and the distance the pulses have to travel in a return journey from the probe to the measured area, in order to avoid a superposition of the returning echo signals. Hence, it is conceivable to increase the pulse-repetition rate when insonifying the ROI, e.g. if the ROI region is relatively close to the US probe, as long as the transducer does not receive signals from different insonifications while listening to the echoes from the ROI.

According to an embodiment, the ROI may be insonified in an alternative measurement mode, e.g. in Doppler mode, i.e. the TX and RX scheme may comprise measuring a Doppler shift in the ROI. This may be done by selecting a Doppler gate, and a specific region where the Doppler shift is measured, i.e. the ROI for which a different transmit/receive scheme is used. In duplex mode, by interlacing the full frame with the Doppler measurements, one can track motion, in particular blood flow, within the ROI and adjust the location of the Doppler gate accordingly. Thereby, one can also use the data reduction method of the invention to dynamically image the anatomical structure, in particular the heart or a portion thereof, in Doppler mode, allowing assessment of e.g. valve function, or other blood vessels. Other alternative measurement modes may comprise strain imaging, such as measuring the deformation rate of a part of an anatomical structure such as the heart muscle, or ultrasound elasticity imaging.

Also, a receive scheme is configured, which is in accordance with the transmit scheme, i.e. configured to receive beams from the regions illuminated with transmit beams.

According to a preferred embodiment, ROI acquisitions are interleaved with full acquisitions of the entire imaging region. In particular, the TX scheme comprises interleaving at least one ROI acquisition cycle, in which only the at least one ROI is insonified, between two full acquisition cycles, in which the entire imaging region of the US probe is insonified; and wherein according to the RX scheme, in a ROI acquisition cycle, the US probe is configured to transfer only the received RF signals corresponding to a depth range of the at least one ROI for at least some of the stipulated receive beams reflected from the at least one ROI, and, in a full acquisition cycle, the US probe is configured to transfer the received RF signals corresponding to the entire imaging region of the US probe. For example, 1-8, preferably 2-4 ROI acquisition cycles may be interleaved between two full acquisition cycles.

According to a preferred embodiment, the US probe comprises an in-probe memory, wherein the in-probe memory is in particular integrated with the ADC array. The method further comprises the steps of selecting the digitized RF data corresponding to a depth range of the at least one ROI for at least some of the stipulated receive beams reflected from the at least one ROI for transfer to the data processing unit, buffering at least some of the selected digitized RF data in the in-probe memory in order to delay the transfer of RF data from the US probe when a pre-determined maximum data rate is exceeded. In some embodiments, a constant output data rate is thereby achieved. The ADC array preferably corresponds to the transducer array. A digital ultrasound probe can easily create more data than can be transferred from the probe to the system (DPU), e.g. if the ADC's sample at a frequency of 12-40 MHz, resulting in a required data transfer rate of up to 50 Gbps, whereas the data link speed, determined by the bandwidth-limited channel, may be only 3-6 Gbps. Accordingly, the data connection between the probe and the DPU may be the limiting factor in many systems. Therefore, it is important to use the available maximum data rate of the interface as well as possible. Accordingly, this embodiment of carrying out the invention involves selecting the relevant RF data, and to discard other digitized RF data, in particular, the RF data corresponding to a depth range outside the ROI region for the beams reflected from the ROI, e.g. in a ROI acquisition cycle. This allows to stream out the relevant RF data, while the US probe is already doing the next acquisitions. In case of insonifying the ROI with higher temporal resolution, the interface should just be able to transfer all digitized (full imaging region and ROI) RF data in a complete cycle comprising one or several ROI acquisition cycles and one full acquisition cycle. However, the interface does not necessarily have to be able to transfer RF data at the native ADC data rate. The in-probe memory is preferably integrated with or in the ADC array, as this is in many cases more energy efficient.

Thus, it is possible to first digitize all received RF data and then select the relevant data corresponding to a depth range of the ROI. Alternatively, it is possible to control the ADC array such that only the receive signals corresponding to a depth range of the ROI for the stipulated transmit beams illuminating the ROI (or the stipulated receive beams reflected from the ROI) are digitized at all. Accordingly, the ADC array may be turned on only in the required depth range. In this embodiment, an in-probe memory may or may not be used, since only the necessary RF data is digitized in the first place, but achieving a constant data rate may still be advantageous.

According to an embodiment, the digitized RF data is processed, in particular compressed before it is transferred to the data processing unit. For example, data compressing methods that can compress dense RF arrays/tenses/buffers, such as Wavelet-compression and/or discrete wavelet transform (DWT), may be used. An example of such compression method would be MPEG data compression protocol.

The digitized RF data from the ROI region and the non-ROI regions may be encoded/processed differently. In particular, the digitized RF data reflected from the at least one ROI may be compressed with a different bit depth, in particular a higher bit-depth, and/or with a different subsampling pattern, e.g. sampling the RF data at a different sampling rate, and/or with a different wavelet compression than the digitized RF data reflected from the imaging region outside the at least one ROI. In particular, the RF data from outside the ROI might be more compressed than data from the ROI in order to decrease the data rate via the limited-bandwidth channel, while at the same time keeping RF data from within one or several ROIs at a sufficiently high quality. It is also conceivable to use a different encoding algorithm for the RF data from the ROI than for the RF data from outside the ROI.

According to an embodiment, the transmit and receive schemes use a wide transmit beam imaging mode, such as a diverging wave, plane wave, or an angled plane wave imaging mode, with a plurality of transmit events to insonify the imaging region. For example, plane wave imaging can be realized by sending 10 - 50 transmit beams in different directions, e.g. 1°-5° transmit beams, from which multiple receive beams will be reconstructed. Only a part of these transmit events insonifies the at least one ROI. Preferably, the transmit events insonifying the at least one ROI are carried out at a higher temporal resolution. For example, in angled plane wave imaging mode, the different transmit beams are in different directions. Depending on the size of the ROI, only a sub-set of the transmit beams are required to image the ROI. In this example, a ROI acquisition cycle will comprise only the sub-set of transmit events, whereas the full acquisition cycle will comprise transmit events in all directions covering the complete azimuth angle, e.g. 90°. According to an embodiment, the transmit power and/or the focusing scheme of transmit beams insonifying the ROI in the TX scheme, in particular in a ROI acquisition cycle, are adapted to the ROI, in particular to the location of the ROI relative to the US probe. For example, the transmit beams can be focused to have the best focus in the depth range of the ROI. Moreover, the transmit power can be adjusted to the distance of the ROI from the US probe, e.g. a higher power is required the longer the distance.

According to an embodiment, two or more regions of interest (ROIs) are selected within the scouting image, the two or more ROIs each including a portion of the anatomical structure, wherein in the transmit (TX) scheme and the receive (RX) scheme the two or more ROIs are insonified with different TX and RX settings than the imaging region of the US probe outside the two or more ROIs. According to an embodiment, the at least two different ROIs are insonified with different TX and RX settings with respect to each other. In other words, different TX and RX settings are applied for the insonification of each ROI from a selection of two or more different ROIs with various grades or aspects of interest, e.g. very high level - medium level - low level. This allows, for example, to discriminate between several different parts of an anatomical structure that each have different properties, e.g. a different movement speed, or are of different importance for a planned medical analysis and therefore require different measurement settings, e.g. different temporal resolutions, in order to image them adequately.

The digitized RF data are preferably blended together into a series of images (video stream) showing the ROI region with a higher temporal resolution than the non-ROI regions. According to an embodiment, this further comprises the step of temporally upsampling the RF data acquired from the imaging region outside the ROI to the temporal resolution of the RF data acquired from the ROI. In case of one or several ROI acquisition cycles interleaved with a full acquisition cycle, one may first beamform the images to reconstruct ROI images and full US images. The full images are then upsampled temporally to the same frame rate as the ROI frame rate. In a next step, the temporally upsampled full US images are blended together with the ROI images, in order to create a sequence of full US images at the frame rate of the ROI images. In one embodiment, a simple linear temporal upsampling/interpolation method may be used, wherein the missing data in-between two full US images are obtained by either duplicating the data from the available full US images, or applying a linear interpolation from the next neighbours. To blend the images together, a simple mask-based blending method may be applied. Preferably, the blending is done in floating point, i.e. with soft mask boundaries. For example, a linear or radial gradient may be applied at the boundaries in order to blend the images. Therein, a mask corresponding to the ROI region is defined within the imaging region, and the ROI images are copied into this mask in the upsampled full US images. According to this embodiment, the blending is performed in the image domain, either in the Cartesian image plane, or in the polar plane, e.g. r-theta. If the full images and the ROI images are in polar coordinates when they are blended together, preferably the blending will happen after the beamforming and before the scan-conversion.

According to an alternative embodiment, the blending step may be done not on the beamformed images, but in the RF domain.

According to an embodiment, the upsampling interpolation may be more complex, in that it takes into account a motion between the at least one ROI and the non-ROI region. This will help to compensate for object motion, such as motion of an anatomical structure and/or its parts, and/or patient motion in-between the plurality of acquisition cycles. Such an algorithm is for example known as Philips natural motion, which is used in video streaming technology to make motion appear more fluent. Alternatively, AI methods, e.g. based on neural networks, may be used. For example, a method as explained in https://heartbeat.fritz.ai/research-guide-for-video-frame-interpolation-with-deep-leamins-519ab2eb3dda or in SuperSloMo: High Quality Estimation of Multiple Intermediate Frames for Video Interpolation, Huaizu Jiang, Dequing Sun, Varun Jampani, Ming-Hsuan Yang, Erik Learned-Miller, Jan Kautz, https:/arxiv.org/abs/1712.00080v2 may be used.

When performing ROI acquisition cycles, according to an embodiment the transmit events insonifying the ROI should start at the same time offset as in the full acquisition cycles. In other words, if the entire imaging region requires a plurality of transmit events, and only a part of the transmit events insonifies the ROI in a ROI acquisition cycle, similar (spatially equivalent) transmit events, i.e. the transmit events in the same direction, are initiated at the same time offset in a ROI acquisition cycle than a full acquisition cycle. This is advantageous, because it makes the blending relatively simple, as the ROI and non-ROI insonifications are generated at a regular time-interleaved manner. Furthermore, it may prevent motion judder, which may otherwise arise due to a discrepancy between the frame rate of a created video sequence and the frame rate of the actual measurement, in particular if the offset between measured frames is irregularly varied. Thus, constant time offsets, i.e. uniform intervals between corresponding transmit events, may provide a smooth motion.

According to an embodiment, the at least one ROI and the imaging region outside the ROI are insonified by overlapping transmit beams, wherein more transmit beams overlap in the at least one ROI than in the non-ROI regions. In other words, one uses large overlapping transmit beams, e.g. such that only 3-8 transmit events are necessary to insonify the complete imaging region. This results in much higher temporal resolution in the zone where all transmit beams overlap, than at the edges. By adjusting the transmit patterns to give most overlap around the ROI zone, an improved (temporal) sampling of the ROI is obtained.

According to an embodiment, the at least one ROI may be detected and tracked automatically by a computer vision or AI algorithm. This may be done on the scouting US image(s), but particularly on the plurality of US images acquired using the method itself, so that the position of the at least one ROI may be automatically adjusted.

The invention is also directed to a US imaging system adapted to carry out the method according to the invention. The ultrasound system may be adapted for ultrafast imaging. However, the invention is not limited to the use for ultrafast US systems, but can be applied also for average speed and/or low cost systems. The invention is generally suitable to improve the imaging quality and/or framerate in a ROI within an imaging region. The system comprises a US probe having a transducer array and an ADC array and being configured to insonify an imaging region, receive echo signals, digitize the received echo signals and transfer the digitized RF data to a DPU, wherein the DPU is adapted to beamform and optionally process the RF data. The US probe of the invention is preferably a high-end probe, e.g. a probe having appropriate integrated circuits for digitization. The invention allows to realize ultrafast imaging at low power consumption of up to 2-3 W. The transducer array may be a 1D or 2D (matrix) array, for example a linear array or phased array with 32-128 transducer elements (channels). The US probe is equipped for in-probe digitization, i.e. the transducer elements are connected to an array of ADCs. Optionally, an in-probe memory is available to temporally store the digitized RF data from the ADCs. According to an embodiment, the array of ADCs are connected to an encoder, and the encoder is adapted to stream the RF data through a data interface, e.g. a serial data link and/or a bandwidth-limited channel, to a decoder connected to the DPU, which may either be part of the US probe, but preferably is outside the US probe and may be part of the main control hardware of the US system. Such control hardware may also comprise a graphical user interface (GUI) allowing the user to view the plurality of ultrasound images and possibly select the ROI, where required.

The DPU is adapted to beamform the digitized RF data. Before or after beamforming, the blending of the ROI acquisitions and the non-ROI acquisitions may be performed. The ADCs, in-probe memory and encoder may be realized by one or several integrated circuits. Also, the decoder and the DPU may be realized by integrated circuits.

SHORT DESCRIPTION OF THE FIGURES

Useful embodiments of the invention shall now be described with reference to the attached figures. Similar elements or features are designated with the same reference signs in the figures. Different embodiments shown are explicitly allowed to be combined unless noted otherwise.

Fig. 1 schematically shows a full ultrasound (US) image with a marked region of interest,
Fig. 2 shows a schematic illustration of an angled plane wave imaging mode with a region of interest according an embodiment of the invention,
Fig. 3 shows a schematic view of an alternative angled plane wave imaging mode with a region of interest according an embodiment of the invention,
Fig. 4 shows an illustration of a receive scheme according to an embodiment of the invention,
Fig. 5 shows a flow diagram representing a method according to an embodiment of the invention,
Fig. 6 shows a schematic illustration of a system according to an embodiment of the invention,
Fig. 7 and 8 show further flow diagrams of a method according to an embodiment of the invention,
Fig. 9 shows a concept of interleaving ROI acquisitions with full acquisitions according to an embodiment of the invention based on data taken with an imaging mode similar to that shown in Fig. 2,
Fig. 10 shows a concept of upsampling full US images to the same frame rate as the ROI frame rate based on data as it is shown in Fig. 9,
Fig. 11 shows a concept of interleaving ROI acquisitions with full acquisitions according to an embodiment of the invention based on data taken with an imaging mode shown in figure 3,
Fig. 12 shows another schematic representation of an ultrasound system according to an embodiment of the invention.

DESCRIPTION OF EMBODIMENTS

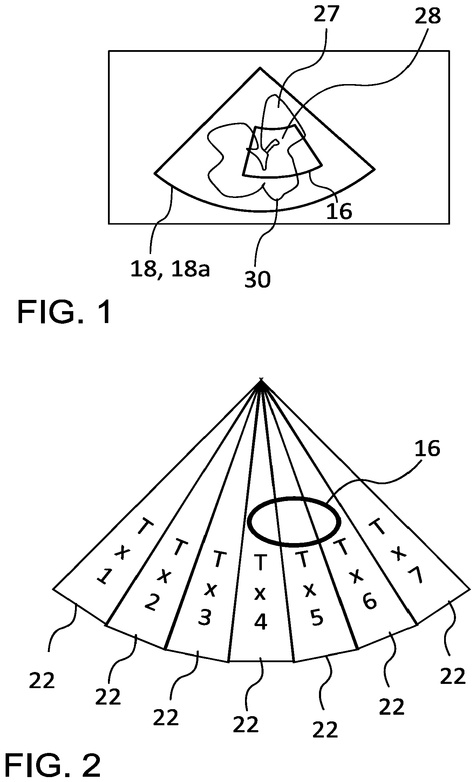

Fig. 1 schematically shows a full ultrasound (US) image 18a corresponding to the imaging region of an US probe. The US image 18a shows a 4-chamber-view of a human heart 30. Within this full US image 18a a region of interest (ROI) 16 is marked. Some parts of the heart, like the valves, move faster than other parts, like the ventricle 27 or atrial walls. In order to resolve the fast movement of the valve 28, it is necessary to apply a sufficiently fast imaging technique, e.g. ultrafast US imaging. On the other hand, other parts of the anatomical structure do not require such a fast imaging technique. Hence, after selecting an ROI 16, which in this example comprises the fast-moving valve 28, the region inside the ROI 16 may be imaged with a higher frame rate than the region outside the ROI 16. For the other parts of the organ 30, the corresponding transmissions of the US probe 10 are scheduled at larger intervals to match the speed of their (slower) movements. Consequently, there will be a slow-time and fast-time acquisition. Such an adaptive TX-RX scheme helps in reducing the radiofrequency (RF) data transfer load compared to a fixed TX-RX scheme, while still allowing to acquire data of the fast-moving valve 28 with a sufficiently high frame rate.

Fig. 2 shows a schematic illustration of an angled plane wave imaging mode according to the invention, wherein different transmit events, i.e. different transmit beams 22, numbered TX1, TX2, TX3, ..., TX7, are directed in different directions, each transmit beam spanning e.g. 5° to 15° of the azimuth angle. In this embodiment the transmit beams 22 are propagating such that consecutive transmit beams 22 are adjacent to one another, and every sector of a predetermined area corresponding to the full imaging region 18 is covered by one transmit beam. In this example, only the transmit beams TX4, TX5 and TX6 are covering the ROI 16. Hence, only 3 out of 7 transmit beams 22 are required to image the ROI 16.

The transmit and receive parameters may be different for the insonification of the ROI than for the full imaging region. For example, the transmit power and/or the transmit focusing scheme may be adapted depending on the location of the ROI 16. Focused TX may help to do harmonic imaging in the ROI 16. Furthermore, the transmit beams 22 that cover the ROI 16 (TX4-TX6) may be transmitted with a higher frequency than the remaining transmit beams 22 of the imaging region 18.

Fig. 3 shows a schematic view of an alternative angled plane wave TX scheme according an embodiment of the invention. In this embodiment, there are three transmit events (TX1, TX2 and TX3) which overlap partially. This results in a much higher temporal resolution in the zone where all transmit beams 22 overlap, than at the edges, which are covered by only one transmit beam 22 (TX1 and TX3 respectively). By adjusting the transmit pattern to provide most overlap around the ROI 16 zone, an improved sampling of the ROI 16 is obtained. In this example, the central zone comprising the ROI 16 is imaged three times more often, namely by all three transmit beams 22 (TX1-TX3) than the two zones at the edges.

Fig. 4 shows an illustration of a receive scheme according to an embodiment of the invention. The receive scheme can be adjusted to only keep samples in the depth range corresponding to the ROI 16 of the stipulated transmit beams 22. In this illustration the full imaging region 18 is represented by a large cube, wherein the horizontal axis is a depth axis (also called fast-time axis), the vertical axis Tx corresponds to the transmit events and the third axis Rx corresponds to the receive elements. The ROI 16 is represented by a smaller cube within the larger cube. In order to reduce the RF data 14 that is forwarded to an analysis system 40 during an ROI acquisition cycle, only the RF data 14 corresponding to a certain depth range, in this case the range from $d_{start}$ to $d_{stop}$ is forwarded. Furthermore, RF data 14 is only forwarded when transmitting into certain transmit angles that cover the ROI 16. In Fig. 4, this range of transmit angles is between $tx_{start}$ and $tx_{stop}$. Following this scheme, the amount of RF data 14 can be reduced by:

$$R_{frame} = \left( \frac{d_{stop} - d_{start}}{d_{full}} \right) \left( \frac{tx_{stop} - tx_{start}}{tx_{full}} \right)$$

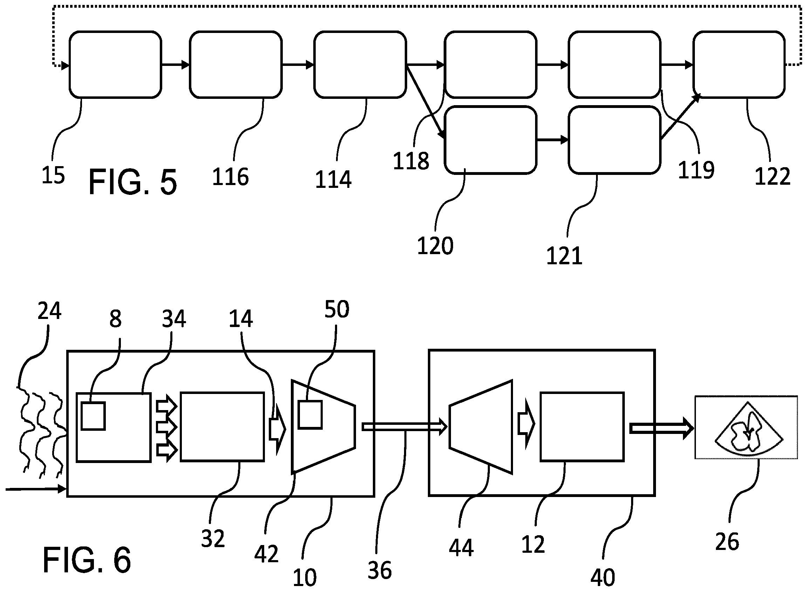

Fig. 5 shows a diagram representing a method according to an embodiment of the invention. As a first step a scouting US image 15 of an anatomical structure of a patient, for example a heart, is acquired. In order to limit the data rates involved, the scouting US image 15 may have lower resolution or may support lower frame rates than images that are acquired later for the actual data analysis. As a next step, an ROI is selected 116 within the scouting US image 15. The ROI 16 may in particular cover a fast-moving portion of an anatomical structure like the valve 28 of a heart. The selection of the ROI 16 may for example be carried out manually by a user or it may be detected automatically by an AI.

Next, a transmit scheme and a receive scheme are adapted for the US imaging system 114 based on the location of the ROI 16 relative to a US probe 10 of the system. The transmit scheme may, for example, comprise insonifying the ROI with a higher temporal resolution, and/or with different types of transmit beams 22 than the imaging region 18 outside the ROI 16. On the other hand, the receive scheme may be according to the embodiment shown in Fig. 4, i.e. at predetermined time intervals corresponding to the transmit scheme only received RF data 14 corresponding to a depth range of the ROI 16 are processed. It is also conceivable to apply a different bit-depth in the ROI 16, e.g. 12-bit precision, than in the region outside the ROI 16, e.g. 8-bit precision, or to measure a Doppler shift within the ROI 16 but not outside of the ROI 16. The following steps are to acquire RF full frame data 118 and ROI frame data 120, wherein according to the transmit scheme, the ROI 16 may for example be insonified with a higher temporal resolution than the imaging region 18 outside the ROI 16. This may be realized by interleaving one or several ROI acquisition cycles, in which only the ROI 16 is insonified, between two full acquisition cycles, in which the entire imaging region 18 is insonified. Hence, for every acquired RF full frame dataset 118 at least one other dataset comprising only the ROI frame is also acquired 120. According to the receive scheme, in an ROI acquisition cycle the US probe 10 is, configured to transfer only the received RF signals corresponding to a depth range of the ROI 16 for the stipulated receive beams 24 reflected from the ROI 16 and in a full acquisition cycle the US probe 10 is configured to transfer the received RF signals corresponding to the entire imaging region 18.

The following steps are to beamform the full frame data 119 and the ROI frame data 121. The ROI images 16a and the full US images 18a are then blended together in a sequence of images to create a temporal sequence of the imaged region, i.e. a video. Thus, a series of US images 26 is created. Optionally, it is conceivable to use one or several of the blended images as a further scouting US image 15 in order to adjust the ROI 16 to accommodate relative movement between the US probe 10 and ROI 16. Accordingly, the steps of this method may be repeated in a cyclical manner, in order to create a plurality of images, wherein the position of the ROI 16 is constantly adjusted.

Fig. 6 shows a schematic illustration of a system according to an embodiment of the invention. The system is configured to acquire a scouting US image 15 of an anatomical structure and automatically or manually select an ROI 16, including a portion of the anatomical structure, within the scouting US image 15. Additionally, the system is configured to set a transmit and receive scheme based on the acquired scouting US image 15 and the selected ROI 16. The system comprises receive elements 34, which include a transducer array 8 that is adapted to emit transmit beams 22 and, as it is shown in Fig. 6, to detect receive beams 24.

Furthermore, an analogue-to-digital converter (ADC), or preferably an array of ADCs 32, one for each transducer element, is provided. The ADC is adapted to digitize the received echo signals and forward them as RF data 14 to the encoder 42. The encoder 42 may for example be an application specific integrated circuit (ASIC) that comprises an in-probe memory 50 which is adapted to temporarily store the relevant data, such as the RF data corresponding to the depth range of the ROI according to a receive scheme as described herein. The encoder 42 is configured to forward the selected RF data 14 via an interface 36 to an analysis system 40. According to the receive scheme set by the system, the encoder 42 is adapted to forward only the received RF data 14 corresponding to a depth range of the ROI 16 for at least some of the stipulated receive beams 24 reflected from the ROI 16, in particular those receive beams 24 corresponding to transmit beams 22 of a ROI acquisition cycle. The encoder 42 is furter configured to transfer the received RF data 14 corresponding to the full depth of the imaging region 18 for at least some of the receive beams 24 reflected from the FOV outside the ROI 16, in particular those receive beams 24 corresponding to transmit beams 22 of a full acquisition cycle.

Since the interface 36 may have a limited capacity concerning the transfer of data, the encoder 42 is preferably adapted to use an in-probe memory 50 as a buffer, in order to control and equalize the data stream forwarded via the interface 36. For example, in case that the ROI 16 is imaged with a higher frequency than the region outside of the ROI 16, the amount of data to be transferred may vary depending on time and may be larger whenever the full imaging region 18 is imaged compared to when only the ROI 16 is imaged. In order to balance this out, the data may be buffered by holding data back, i.e. storing some of the generated RF data 14 on the in-probe memory 50, when a large amount of RF data 14 is generated, and forwarding the stored data, when an altogether lower amount of RF data 14 is generated, together with the lower amount of currently generated RF data 14. Thus, an even stream of data without any data peaks will be forwarded. Preferably, all the relevant data can be transferred within one complete cycle that comprises both at least one ROI acquisition cycle and one full acquisition cycle with a relatively constant data stream, wherein the data rate does in particular not exceed a predetermined threshold. Advantageously, the encoder 42 may further comprise means to compress RF data 14, e.g. wavelet compression, to achieve a further minimization of the forwarded data.

The system furthermore comprises an analysis system 40 including a decoder 44 that is configured to receive the transferred RF data 14, possibly decompress it, and provide it to a data processing unit 12 which includes a beamformer. By utilizing the data processing unit 12, the analysis system 40 is adapted to process the RF data 14 and create US images 26.

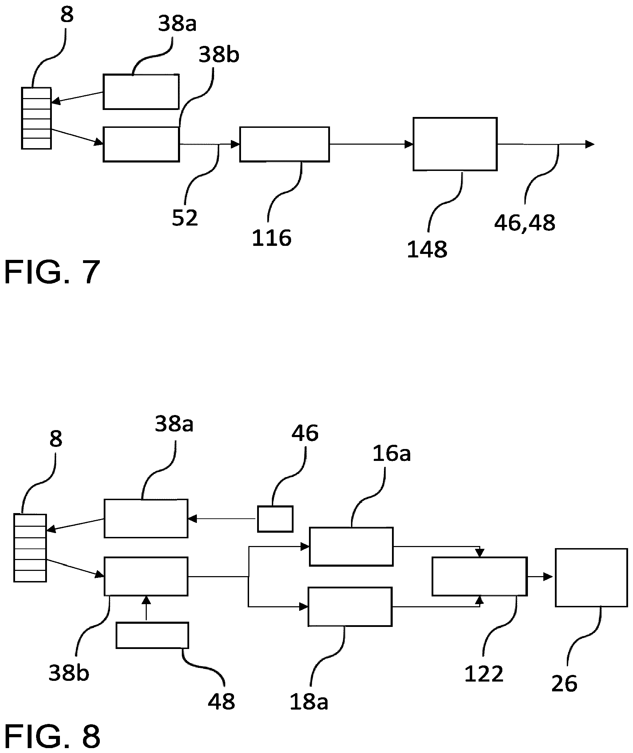

Figs. 7 and 8 show another schematic representation of a method according to an embodiment of the invention. Fig. 7 represents the first stage, in which the ROI 16 is selected and corresponding TX and RX schemes are set. Therein, first a TX beamforming pattern 38a is transmitted to a transducer array 8. The transducer array 8 emits transmit beams 22 and detects corresponding receive beams 24, which are digitized and processed via RX beamforming 38b. Corresponding B-mode images 52 are then used to select an ROI 16. According to the selected ROI 16, corresponding TX and RX schemes 46, 48 are then selected 148 and redirected to the transducer array 8. As it is shown in Fig. 8 the TX scheme 46 is then used for TX beamforming 38a via the transducer array 8 and incoming signals received by the transducer array 8 are digitized and processed via RX beamforming 38b according to the set RX scheme 48. According to this RX scheme 48, one or several ROI images 16a and a full US image 18a of the imaging region 18 are generated. These images are then blended in the final step 122 in order to create a series of US images 26.

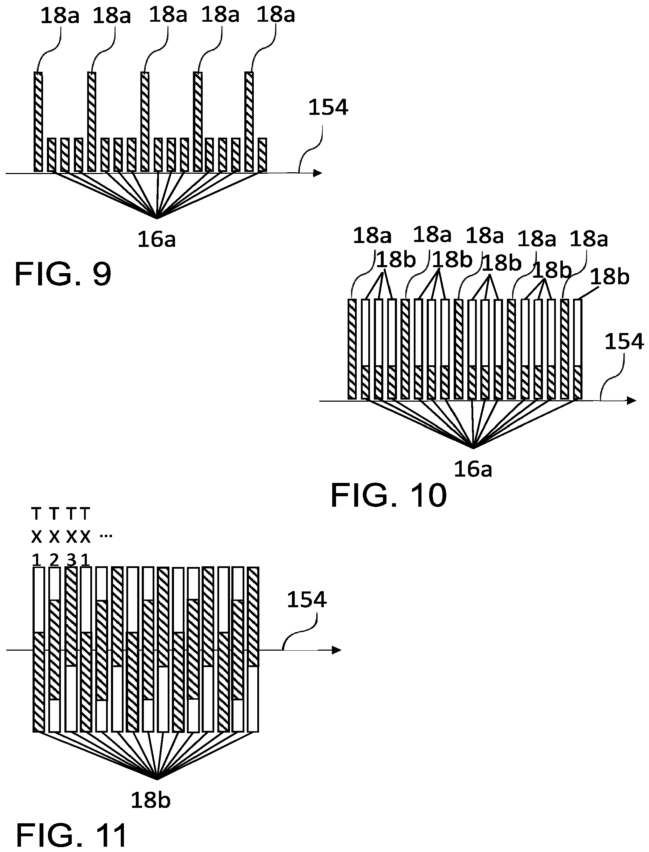

Fig. 9 shows a concept of interleaving ROI acquisitions with full acquisitions according to an embodiment of the invention. The concept is to take images of the ROI 16 at a higher frequency, i.e. take a larger number of ROI images 16a per time, while also keeping the full imaging region 18 imaged but with a lower frequency, i.e. by taking fewer full US images 18a per time. In this embodiment, there are three ROI images 16a for every full US image 18a. A time axis 145 shows the order, in which the images are taken. Hence, after each full US image 18a, three ROI images 16a are consecutively taken, before the next full US image 18a is taken. Preferably, the time offset between corresponding TX and RX events in each acquired image is constant. This makes it easier to blend the images together and achieve a smooth motion of a corresponding resulting video. Generally, by interlacing one full US image 18a and n-1 ROI images 16a and based on the reduction factor $R_{frame}$ introduced previously the total reduction of data rate may be described by the reduction factor $R_{tot}$:

$$R_{tot} = \frac{(n-1) * R_{frame} + 1}{n}$$

Hence, the total reduction of data rate in the example shown in Fig. 9 is $R_{tot}=(3R_{frame}+1)/4$. Dependent on the size of the specific ROI, a total data reduction factor $R_{tot}$ between 2 and 5 can thus be achieved.

Fig. 10 is based on data as it is shown in Fig. 9, wherein full US images 18a are upsampled to the same frame rate as the ROI frame rate. The hatched parts of the bars correspond to measured RF data 14, while the empty parts of the bars correspond to upsampled data. Upsampling of the full US images 18a may be achieved by a simple linear temporal interpolation method. Hence, interpolated full images 18b based on the previous and the following acquired full US image 18a are generated at time steps corresponding to the time steps of acquired ROI images 16a. Thus, the same number of full imaging region images, comprising interpolated full images 18b and originally measured full US images 18a, is created as there are ROI images 16a. The upsampled full frame images are then blended with the ROI images 16a in order to create a full video sequence of the imaging region 18. The resulting video will have a higher quality in the ROI 16 than in the region outside the ROI 16, i.e. it will be temporally more accurate. It is conceivable that during the interpolation of the images, the motion between the ROI 16 and the region outside of the ROI 16 is taken into account. This may be supported by utilizing a neural network. Furthermore, the blending might be either performed in the image domain as described above, i.e. pixel-based by adding or averaging intensities of two images, or it might also be performed in the RF domain before the actual images are created by using Gaussian intensity profiles, in particular weighted Gaussian intensity profiles. Blending in the image domain might be either done in polar coordinates, which are the natural coordinates that originate from the geometry of the ultrasound image taking system, or blending can be done in Cartesian coordinates that are calculated from the polar coordinates during a scan conversion.

The images as they are represented in Figs. 9 and 10 may for example be acquired in diverging wave or in angled plane imaging mode similar to that shown in Fig. 2. When acquiring images according to an embodiment as shown in Fig. 3, wherein different transmit events overlap, an interleaving scheme as it is shown in Fig. 11 may be applied. Again, the hatched areas of the bars represent actually measured RF data 14 while the empty parts of the bars result from an interpolation of the images. In the central area close to the time axis 154, corresponding to the ROI 16, the image data resulting from all three different transmit beams 22 (TX1, TX2 and TX3) overlap, while on the upper part (TX3) and in the lower part (TX1) of the figure, corresponding to the area outside the ROI 16, only one transmit beam (TX1 and TX3, respectively) contributes to the actual image data. Similar to the embodiments shown in Figs. 9 and 10, the remaining empty spaces may be filled by interpolation, thereby the images are upsampled to show the whole area at every time step. Again, the interpolated full images 18b are then blended together in order to create a video sequence, wherein the ROI 16 is represented temporally more accurate.

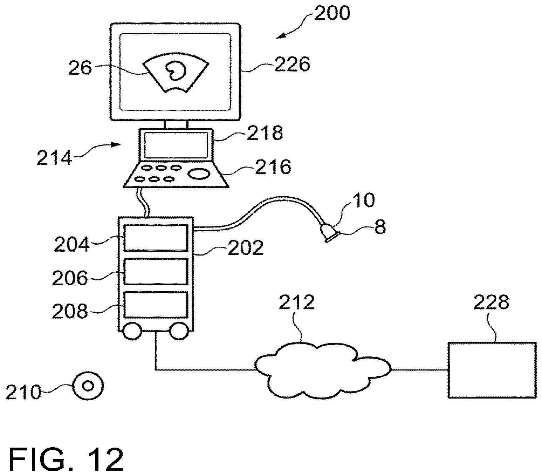

Fig. 12 shows a schematic representation of an ultrasound system 200 according to an embodiment of the invention and configured to perform the inventive method. It may in particular be configured to work as schematically shown in Fig. 5. The ultrasound system 200 includes a usual ultrasound hardware unit 202, comprising a CPU 204, GPU 206 and digital storage medium 208, for example a hard disc or solid-state disc. A computer program may be loaded into the hardware unit, from CD-ROM 210 or over the internet 212. The hardware unit 202 is connected to a user-interface 214, which comprises a keyboard 216 and optionally a touchpad 218. The touchpad 218 may also act as a display device for displaying imaging parameters. The hardware unit 202 is connected to a US probe 10, which includes a transducer array 8 and optionally an in-probe memory 50 (not shown). The US probe 10 is configured to insonify an imaging region, receive echo signals, digitize the received echo signals and transfer the digitized RF data to a data processing unit 12, wherein the data processing unit 12 may be part of the CPU 204 and/or the GPU 206. Acquired US images 26 are displayed on the screen 226, which may be any commercially available display unit, e.g. a screen, television set, flat screen, projector etc.

The above discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended scope of the present invention, as set forth in the claims that follow. Accordingly, the specification and drawings are to be regarded as an illustrative manner and are not intended to limit the scope of the appended claims.

REFERENCE SIGNS

| 8 | transducer array |
|---|---|
| 10 | US probe |
| 12 | data processing unit |
| 14 | RF data |
| 15 | scouting US image |
| 16 | ROI |
| 16a | ROI image |
| 18 | imaging region |
| 18a | Full US image |
| 18b | interpolated full image |
| 22 | transmit beam |
| 24 | receive beam |
| 26 | series of US images |
| 27 | ventricle |
| 28 | valve |
| 30 | heart |
| 32 | ADC array |
| 34 | receive elements |
| 34a | receive element axis |
| 35a | transmit event axis |
| 36 | interface |
| 38 | beamforming |
| 38a | TX beamforming |
| 38b | RX beamforming |
| 40 | analysis system |
| 42 | encoder |
| 44 | decoder |
| 46 | TX scheme |
| 48 | RX scheme |
| 50 | in-probe memory |
| 52 | B-mode |
| 114 | adapt TX/RX scheme |
| 116 | select ROI |
| 118 | acquire RF Full Frame |
| 119 | beamform Full Frame |
| 120 | acquire ROI frame |
| 121 | beamform ROI frame |
| 122 | video/image blending |
| 148 | select TX and RX scheme |
| 154 | time axis |
| 200 | ultrasound system |
| 202 | ultrasound hardware unit |
| 204 | CPU |
| 206 | GPU |
| 208 | digital storage medium |
| 210 | CD-ROM |
| 212 | internet |
| 214 | user-interface |
| 216 | keyboard |
| 218 | touchpad |

226    screen

**Claims**

1. A method for ultrasound (US) imaging using an ultrasound system comprising an US probe (10) having a transducer array (8) and an analogue-to-digital converter (ADC) array (32), wherein the US probe (10) is configured to insonify an imaging region (18), receive echo signals, digitize the received echo signals by the ADC array (32) and transfer the digitized radiofrequency (RF) data (14) to a data processing unit (12) via a bandwidth-limited channel, wherein the data processing unit (12) is adapted to beamform the RF data (14), the method comprising the following steps:

   - acquiring at least one scouting image (15) of the imaging region (18), the scouting image (15) including an anatomical structure of interest;
   - selecting at least one region of interest (ROI) (16) within the scouting image (15), wherein the at least one ROI (16) includes a portion of the anatomical structure;
   - setting a transmit (TX) scheme (46) and a receive (RX) scheme (48) for US imaging of the imaging region (18), wherein the at least one ROI (16) is insonified with different TX and RX settings than the imaging region (18) outside the at least one ROI (16);
   - acquiring a plurality of US images (26) according to the TX scheme (46) and the RX scheme (48);

   wherein the US probe (10) is configured to transfer to the data processing unit (12) only the received RF data (14) corresponding to a depth range of the at least one ROI (16) for at least some of the stipulated receive beams (24) reflected from the at least one ROI (16), and is configured to transfer the received RF data (14) corresponding to the full depth of the imaging region (18) for at least some of the receive beams (24) reflected from the imaging region (18) outside the at least one ROI (16).

2. The method according to claim 1, wherein TX and RX schemes (46, 48) comprise insonifying the at least one ROI (16) with a higher temporal resolution and/or with different types of transmit beams (22) and/or with a different pulse-repetition rate than the imaging region (18) outside the at least one ROI (16).

3. The method according to claim 1 or 2, wherein, in the TX scheme (46), the transmit power and/or the focusing scheme of transmit beams (22) insonifying the at least one ROI (16) are adapted to the at least one ROI (16), in particular to the location of the at least one ROI (16) relative to the US probe (10).

4. The method according to any one of the preceding claims,

   - wherein the TX scheme (46) and RX scheme (48) comprise interleaving at least one ROI acquisition cycle, in which only the at least one ROI (16) is insonified, between two full acquisition cycles, in which the entire imaging region (18) of the US probe (10) is insonified; and
   - wherein according to the RX scheme (48), in a ROI acquisition cycle, the US probe (10) is configured to transfer only the received RF data (14) corresponding to a depth range of the at least one ROI (16) for at least some of the stipulated receive beams (24) reflected from the at least one ROI (16), and, in a full acquisition cycle, the US probe (10) is configured to transfer the received RF data (14) corresponding to the depth range of the entire imaging region (18) of the US probe (10).

5. The method according to any one of the preceding claims, wherein the US probe (10) comprises an in-probe memory (50), wherein the in-probe memory (50) is in particular integrated with the ADC array (32), the method further comprising the steps of:

   - selecting the digitized RF data (14) corresponding to a depth range of the at least one ROI (16) for at least some of the stipulated receive beams (24) reflected from the at least one ROI (16) for transfer to the data processing unit (12);
   - buffering at least some of the selected digitized RF data (14) in the in-probe memory (50) in order to delay the transfer of RF data (14) from the US probe (10) when a pre-determined maximum data rate is exceeded.

6. The method according to any one of the preceding claims, wherein the digitized RF data (14) are compressed, in particular via a discrete wavelet transform, before they are transferred to the data processing unit (12).

7. The method according to claim 6, wherein the digitized RF data (14) reflected from the at least one ROI (16) are compressed with a different bit depth, in particular a higher bit-depth, and/or with a different subsampling pattern and/or with a different wavelet compression than the digitized RF data (14) reflected from the imaging region (18) outside the at least one ROI (16).

8. The method according to any one of the preceding claim, wherein the TX and RX scheme (48) uses a wide transmit beam imaging mode with a plurality of transmit events to insonify the imaging region (18), wherein only a part of the plurality of transmit events insonifies the at least one ROI (16), and wherein the transmit events insonifying the at least one ROI (16) are carried out at a higher temporal resolution.

9. The method according to any one of the preceding claims 4 to 8, wherein the TX scheme (46) uses a plurality of transmit events to insonify the imaging region (18), wherein a part of the plurality of transmit events insonifies the at least one ROI (16), wherein spatially equivalent transmit events are initiated at the same time offset in at least one ROI acquisition cycle and a full acquisition cycle.

10. The method according to any one of the preceding claims,

   - wherein two or more regions of interest (ROIs) (16) are selected within the scouting image (15), the two or more ROIs (16) each including a portion of the anatomical structure,
   - wherein in the TX scheme (46) and the RX scheme, the two or more ROIs (16) are insonified with different TX and RX settings than the imaging region (18) outside the two or more ROIs (16), and
   - wherein at least two different ROIs (16) are insonified with different TX and RX settings with respect to each other.

11. The method according to any one of the preceding claims 3 to 10, further comprising the following steps:

   - reconstructing ROI images (16a) from the ROI acquisition cycle and full US images (18a) from the full acquisition cycles;
   - temporally upsampling the full US images (18a) in order to achieve the same frame rate of full US images (18a) for the entire imaging region (18) as for the ROI images (16a);
   - blending together the upsampled full US images (18a) with the ROI images (16a).

12. The method according to claim 10 or 11, wherein upsampling is carried out using interpolation, and the interpolation takes into account motion between the at least one ROI (16) and the remaining imaging region (18), in particular by using a trained neural network.

13. The method according to any one of the preceding claims, wherein, in the TX scheme (46), the at least one ROI (16) and the imaging region (18) outside the at least one ROI (16) are insonified by overlapping transmit beams (22), wherein more transmit beams (22) overlap in the at least one ROI than in the imaging region (18) outside the at least one ROI (16).

14. The method according to any one of the preceding claims, wherein the at least one ROI (16) is detected and tracked automatically by a computer vision and/or an artificial intelligence algorithm on the at least one scouting image (15) and/or on the plurality of ultrasound images (26).

15. An US imaging system (200) adapted to carry out the method according to any one of the previous claims, the system comprising an US probe (10) having a transducer array (8) and an ADC array (32), wherein the US probe (10) is configured to insonify an imaging region (18), receive echo signals, digitize the received echo signals and transfer the digitized RF data (14) via an interface (36) comprising a bandwidth-limited channel to a data processing unit (12), wherein the data processing unit (12) is adapted to process and beamform the RF data (14).

**Patentansprüche**

1. Verfahren zur Ultraschall (US) Bildgebung unter Verwendung eines Ultraschallsystems, das eine US-Sonde (10) umfasst, die eine Wandleranordnung (8) und eine Analog-Digital-Wandler- (ADC) Anordnung (32) aufweist, wobei die US-Sonde (10) so konfiguriert ist, dass sie einen Bildgebungsbereich (18) beschallt, Echosignale empfängt, die empfangenen Echosignale durch die ADC-Anordnung (32) digitalisiert und die digitalisierten Hochfrequenz- (HF-) Daten (14) über einen bandbreitenbegrenzten Kanal an eine Datenverarbeitungseinheit (12) überträgt, wobei die Datenverarbeitungseinheit (12) so angepasst ist, dass sie die HF-Daten (14) strahlformt, wobei das Verfahren die folgenden Schritte umfasst:

- Erfassen mindestens eines Erkundungsbildes (15) des Bildgebungsbereichs (18), wobei das Erkundungsbild (15) eine anatomische Struktur von Interesse beinhaltet;
- Auswählen mindestens eines Bereichs von Interesse (ROI) (16) innerhalb des Erkundungsbildes (15), wobei der mindestens eine ROI (16) einen Abschnitt der anatomischen Struktur beinhaltet;
- Einstellen eines Sende- (TX) Schemas (46) und eines Empfangs- (RX) Schemas (48) für US-Bildgebung des Bildgebungsbereichs (18), wobei der mindestens eine ROI (16) mit anderen TX- und RX-Einstellungen beschallt wird als der Bildgebungsbereich (18) außerhalb des mindestens einen ROI (16);
- Erfassen einer Vielzahl von US-Bildern (26) gemäß dem TX-Schema (46) und dem RX-Schema (48);

wobei die US-Sonde (10) so konfiguriert ist, dass sie an die Datenverarbeitungseinheit (12) nur die empfangenen HF-Daten (14) überträgt, die einem Tiefenbereich des mindestens einen ROI (16) für mindestens einige der festgelegten Empfangsstrahlen (24), die von dem mindestens einen ROI (16) reflektiert werden, entsprechen, und so konfiguriert ist, dass sie die empfangenen HF-Daten (14) überträgt, die der vollständigen Tiefe des Bildgebungsbereichs (18) für mindestens einige der Empfangsstrahlen (24), die von dem Bildgebungsbereich (18) außerhalb des mindestens einen ROI (16) reflektiert werden, entsprechen.

2. Verfahren nach Anspruch 1, wobei TX- und RX-Schemata (46, 48) das Beschallen des mindestens einen ROI (16) mit einer höheren zeitlichen Auflösung und/oder mit unterschiedlichen Arten von Sendestrahlen (22) und/oder mit einer anderen Pulswiederholrate als der Bildgebungsbereich (18) außerhalb des mindestens einen ROI (16) umfassen.

3. Verfahren nach Anspruch 1 oder 2, wobei im TX-Schema (46) die Sendeleistung und/oder das Fokussierungsschema von Sendestrahlen (22), die den mindestens einen ROI (16) beschallen, an den mindestens einen ROI (16), insbesondere an die Lage des mindestens einen ROI (16) relativ zur US-Sonde (10), angepasst sind.

4. Verfahren nach einem der vorstehenden Ansprüche,

- wobei das TX-Schema (46) und das RX-Schema (48) das Verschachteln mindestens eines ROI-Erfassungszyklus, in dem nur der mindestens eine ROI (16) beschallt wird, zwischen zwei vollständigen Erfassungszyklen, in denen der gesamte Bildgebungsbereich (18) der US-Sonde (10) beschallt wird, umfassen; und
- wobei gemäß dem RX-Schema (48) in einem ROI-Erfassungszyklus die US-Sonde (10) so konfiguriert ist, dass sie nur die empfangenen HF-Daten (14) überträgt, die einem Tiefenbereich des mindestens einen ROI (16) für mindestens einige der festgelegten Empfangsstrahlen (24), die von dem mindestens einen ROI (16) reflektiert werden, entsprechen, und in einem vollständigen Erfassungszyklus die US-Sonde (10) so konfiguriert ist, dass sie die empfangenen HF-Daten (14) überträgt, die dem Tiefenbereich des gesamten Bildgebungsbereichs (18) der US-Sonde (10) entsprechen.

5. Verfahren nach einem der vorstehenden Ansprüche, wobei die US-Sonde (10) einen sondeninternen Speicher (50) umfasst, wobei der sondeninterne Speicher (50) insbesondere in die ADC-Anordnung (32) integriert ist, wobei das Verfahren weiter die folgenden Schritte umfasst:

- Auswählen der digitalisierten HF-Daten (14), die einem Tiefenbereich des mindestens einen ROI (16) für mindestens einige der festgelegten Empfangsstrahlen (24), die von dem mindestens einen ROI (16) reflektiert werden, entsprechen, zur Übertragung an die Datenverarbeitungseinheit (12);
- Puffern mindestens einiger der ausgewählten digitalisierten HF-Daten (14) im sondeninternen Speicher (50), um die Übertragung von HF-Daten (14) von der US-Sonde (10) zu verzögern, wenn eine vorbestimmte maximale Datenrate überschritten wird.

6. Verfahren nach einem der vorstehenden Ansprüche, wobei die digitalisierten HF-Daten (14) komprimiert werden, insbesondere über eine diskrete Wavelet-Transformation, bevor sie an die Datenverarbeitungseinheit (12) übertragen werden.

7. Verfahren nach Anspruch 6, wobei die digitalisierten HF-Daten (14), die von dem mindestens einen ROI (16) reflektiert werden, mit einer anderen Bittiefe, insbesondere einer höheren Bittiefe, und/oder mit einem anderen Unterabtastungsmuster und/oder mit einer anderen Wavelet-Kompression komprimiert werden als die digitalisierten HF-Daten (14), die von dem Bildgebungsbereich (18) außerhalb des mindestens einen ROI (16) reflektiert werden.

8. Verfahren nach einem der vorstehenden Ansprüche, wobei das TX- und RX-Schema (48) einen Bildgebungsmodus mit breitem Sendestrahl mit einer Vielzahl von Sendeereignissen verwendet, um den Bildgebungsbereich (18) zu beschallen, wobei nur ein Teil der Vielzahl von Sendeereignissen den mindestens einen ROI (16) beschallt, und wobei die Sendeereignisse, die den mindestens einen ROI (16) beschallen, mit einer höheren zeitlichen Auflösung ausgeführt werden.

9. Verfahren nach einem der vorstehenden Ansprüche 4 bis 8, wobei das TX-Schema (46) eine Vielzahl von Sendeereignissen verwendet, um den Bildgebungsbereich (18) zu beschallen, wobei ein Teil der Vielzahl von Sendeereignissen den mindestens einen ROI (16) beschallt, wobei räumlich äquivalente Sendeereignisse mit dem gleichen Zeitversatz in mindestens einem ROI-Erfassungszyklus und einem vollständigen Erfassungszyklus initiiert werden.

10. Verfahren nach einem der vorstehenden Ansprüche,

- wobei zwei oder mehr Bereiche von Interesse (ROIs) (16) innerhalb des Erkundungsbildes (15) ausgewählt werden, wobei die zwei oder mehr ROIs (16) jeweils einen Abschnitt der anatomischen Struktur beinhalten,
- wobei in dem TX-Schema (46) und dem RX-Schema die zwei oder mehr ROIs (16) mit anderen TX- und RX-Einstellungen beschallt werden als der Bildgebungsbereich (18) außerhalb der zwei oder mehr ROIs (16), und
- wobei mindestens zwei verschiedene ROIs (16) mit unterschiedlichen TX- und RX-Einstellungen in Bezug aufeinander beschallt werden.

11. Verfahren nach einem der vorstehenden Ansprüche 3 bis 10, das weiter die folgenden Schritte umfasst:

- Rekonstruieren von ROI-Bildern (16a) aus dem ROI-Erfassungszyklus und vollständigen US-Bildern (18a) aus den vollständigen Erfassungszyklen;
- zeitliches Upsampling der vollständigen US-Bilder (18a), um für den gesamten Bildgebungsbereich (18) die gleiche Bildrate der vollständigen US-Bilder (18a) wie für die ROI-Bilder (16a) zu erreichen;
- Zusammenführen der upgesampelten vollständigen US-Bilder (18a) mit den ROI-Bildern (16a).

12. Verfahren nach Anspruch 10 oder 11, wobei das Upsampling unter Verwendung von Interpolation ausgeführt wird und die Interpolation Bewegung zwischen dem mindestens einen ROI (16) und dem übrigen Bildgebungsbereich (18) berücksichtigt, insbesondere unter Verwendung eines trainierten neuronalen Netzwerks.

13. Verfahren nach einem der vorstehenden Ansprüche, wobei im TX-Schema (46) der mindestens eine ROI (16) und der Bildgebungsbereich (18) außerhalb des mindestens einen ROI (16) durch überlappende Sendestrahlen (22) beschallt werden, wobei sich in dem mindestens einen ROI mehr Sendestrahlen (22) überlappen als in dem Bildgebungsbereich (18) außerhalb des mindestens einen ROI (16).

14. Verfahren nach einem der vorstehenden Ansprüche, wobei der mindestens eine ROI (16) automatisch durch einen Computervisions- und/oder einen Künstliche-Intelligenz-Algorithmus auf dem mindestens einen Erkundungsbild (15) und/oder auf der Vielzahl von Ultraschallbildern (26) erkannt und verfolgt wird.

15. US-Bildgebungssystem (200), das so angepasst ist, dass es das Verfahren nach einem der vorstehenden Ansprüche ausführt, wobei das System eine US-Sonde (10) umfasst, die eine Wandleranordnung (8) und eine ADC-Anordnung (32) aufweist, wobei die US-Sonde (10) so konfiguriert ist, dass sie einen Bildgebungsbereich (18) beschallt, Echosignale empfängt, die empfangenen Echosignale digitalisiert und die digitalisierten HF-Daten (14) über eine Schnittstelle (36), die einen bandbreitenbegrenzten Kanal umfasst, an eine Datenverarbeitungseinheit (12) überträgt, wobei die Datenverarbeitungseinheit (12) so angepasst ist, dass sie die HF-Daten (14) verarbeitet und strahlformt.

**Revendications**

1. Procédé d'imagerie à ultrasons (US) utilisant un système à ultrasons comprenant une sonde US (10) présentant un réseau de transducteurs (8) et un réseau de convertisseurs analogique-numérique (ADC) (32), dans lequel la sonde US (10) est configurée pour insonifier une région d'imagerie (18), recevoir des signaux d'écho, numériser les signaux d'écho reçus par l'intermédiaire du réseau ADC (32) et transférer les données radiofréquence (RF) numérisées (14) à une unité de traitement de données (12) via un canal limité en bande passante, dans lequel l'unité de traitement de données (12) est adaptée pour former en faisceau les données RF (14), le procédé comprenant les étapes suivantes :

- l'acquisition d'au moins une image de repérage (15) de la région d'imagerie (18), l'image de repérage (15) incluant une structure anatomique d'intérêt ;
- la sélection d'au moins une région d'intérêt (ROI) (16) au sein de l'image de repérage (15), dans lequel la au moins une ROI (16) inclut une partie de la structure anatomique ;
- la définition d'un schéma d'émission (TX) (46) et d'un schéma de réception (RX) (48) pour l'imagerie US de la région d'imagerie (18), dans lequel la au moins une ROI (16) est insonifiée avec des réglages TX et RX différents de la région d'imagerie (18) à l'extérieur de la au moins une ROI (16) ;
- l'acquisition d'une pluralité d'images US (26) selon le schéma TX (46) et le schéma RX (48) ;

dans lequel la sonde US (10) est configurée pour transférer à l'unité de traitement de données (12) uniquement les données RF reçues (14) correspondant à une plage de profondeur de la au moins une ROI (16) pour au moins certains des faisceaux de réception stipulés (24) réfléchis par la au moins une ROI (16), et est configurée pour transférer les données RF reçues (14) correspondant à toute la profondeur de la région d'imagerie (18) pour au moins certains des faisceaux de réception (24) réfléchis par la région d'imagerie (18) à l'extérieur de la au moins une ROI (16).

2. Procédé selon la revendication 1, dans lequel les schémas TX et RX (46, 48) comprennent l'insonification de la au moins une ROI (16) avec une résolution temporelle plus élevée et/ou avec différents types de faisceaux d'émission (22) et/ou avec un taux de répétition d'impulsions différent de la région d'imagerie (18) à l'extérieur de la au moins une ROI (16).

3. Procédé selon la revendication 1 ou 2, dans lequel, dans le schéma TX (46), la puissance d'émission et/ou le schéma de focalisation des faisceaux d'émission (22) insonifiant la au moins une ROI (16) sont adaptés à la au moins une ROI (16), en particulier à l'emplacement de la au moins une ROI (16) par rapport à la sonde US (10).

4. Procédé selon l'une quelconque des revendications précédentes,

- dans lequel le schéma TX (46) et le schéma RX (48) comprennent un entrelacement d'au moins un cycle d'acquisition de ROI, dans lequel seule la au moins une ROI (16) est insonifiée, entre deux cycles d'acquisition complets, dans lesquels la région d'imagerie entière (18) de la sonde US (10) est insonifiée ; et
- dans lequel selon le schéma RX (48), dans un cycle d'acquisition de ROI, la sonde US (10) est configurée pour transférer uniquement les données RF reçues (14) correspondant à une plage de profondeur de la au moins une ROI (16) pour au moins certains des faisceaux de réception stipulés (24) réfléchis par la au moins une ROI (16), et, dans un cycle d'acquisition complet, la sonde US (10) est configurée pour transférer les données RF reçues (14) correspondant à la plage de profondeur de toute la région d'imagerie (18) de la sonde US (10).

5. Procédé selon l'une quelconque des revendications précédentes, dans lequel la sonde US (10) comprend une mémoire intégrée à la sonde (50), dans laquelle la mémoire intégrée à la sonde (50) est en particulier intégrée au réseau ADC (32), le procédé comprenant en outre les étapes suivantes :

- la sélection des données RF numérisées (14) correspondant à une plage de profondeur de la au moins une ROI (16) pour au moins certains des faisceaux de réception stipulés (24) réfléchis par la au moins une ROI (16) pour transfert à l'unité de traitement de données (12) ;
- la mise en tampon d'au moins certaines des données RF numérisées (14) sélectionnées dans la mémoire intégrée à la sonde (50) afin de retarder le transfert de données RF (14) depuis la sonde US (10) lorsqu'un débit de données maximum prédéterminé est dépassé.

6. Procédé selon l'une quelconque des revendications précédentes, dans lequel les données RF numérisées (14) sont compressées, notamment via une transformée en ondelettes discrète, avant d'être transférées à l'unité de traitement de données (12).

7. Procédé selon la revendication 6, dans lequel les données RF numérisées (14) réfléchies par la au moins une ROI (16) sont compressées avec une profondeur de bit différente, en particulier une profondeur de bit plus élevée, et/ou avec un modèle de sous-échantillonnage différent et/ou avec une compression d'ondelettes différente de celle des données RF numérisées (14) réfléchies par la région d'imagerie (18) à l'extérieur de la au moins une ROI (16).

8. Procédé selon l'une quelconque des revendications précédentes, dans lequel le schéma TX et RX (48) utilise un mode d'imagerie à large faisceau d'émission avec une pluralité d'événements d'émission pour insonifier la région d'imagerie (18), dans lequel seule une partie de la pluralité d'événements d'émission insonifie la au moins une ROI (16), et dans lequel les événements d'émission insonifiant la au moins une ROI (16) sont réalisés à une résolution temporelle plus élevée.

9. Procédé selon l'une quelconque des revendications précédentes 4 à 8, dans lequel le schéma TX (46) utilise une pluralité d'événements d'émission pour insonifier la région d'imagerie (18), dans lequel une partie de la pluralité d'événements d'émission insonifie la au moins une ROI (16), dans lequel des événements d'émission spatialement équivalents sont initiés en même temps décalés dans au moins un cycle d'acquisition de ROI et un cycle d'acquisition complet.

10. Procédé selon l'une quelconque des revendications précédentes,

- dans lequel deux régions d'intérêt (ROI) (16) ou plus sont sélectionnées dans l'image de repérage (15), les deux ROI (16) ou plus incluant chacune une partie de la structure anatomique,
- dans lequel dans le schéma TX (46) et le schéma RX, les deux ROI (16) ou plus sont insonifiées avec des paramètres TX et RX différents de la région d'imagerie (18) à l'extérieur des deux ROI (16) ou plus, et
- dans lequel au moins deux ROI (16) différentes sont insonifiées avec des réglages TX et RX différents l'un par rapport à l'autre.

11. Procédé selon l'une quelconque des revendications précédentes 3 à 10, comprenant en outre les étapes suivantes :

- la reconstruction des images de ROI (16a) à partir du cycle d'acquisition de ROI et des images US complètes (18a) à partir des cycles d'acquisition complets ;
- le suréchantillonnage temporel des images US complètes (18a) afin d'obtenir la même fréquence de trames d'images US complètes (18a) pour la région d'imagerie entière (18) que pour les images ROI (16a) ;
- le mélange des images US complètes (18a) suréchantillonnées avec les images de ROI (16a).

12. Procédé selon la revendication 10 ou 11, dans lequel le suréchantillonnage est effectué à l'aide d'une interpolation, et l'interpolation prend en compte un mouvement entre la au moins une ROI (16) et la région d'imagerie (18) restante, en particulier en utilisant un réseau neuronal entraîné.

13. Procédé selon l'une quelconque des revendications précédentes, dans lequel dans le schéma TX (46), la au moins une ROI (16) et la région d'imagerie (18) à l'extérieur de la au moins une ROI (16) sont insonifiées par des faisceaux d'émission en chevauchement (22), dans lequel davantage de faisceaux d'émission (22) se chevauchent dans la au moins une ROI que dans la région d'imagerie (18) à l'extérieur de la au moins une ROI (16).

14. Procédé selon l'une quelconque des revendications précédentes, dans lequel la au moins une ROI (16) est détectée et suivie automatiquement par une vision informatique et/ou un algorithme d'intelligence artificielle sur la au moins une image de repérage (15) et/ou sur la pluralité d'images ultrasonores (26).

15. Système d'imagerie US (200) adapté pour mettre en oeuvre le procédé selon l'une quelconque des revendications précédentes, le système comprenant une sonde US (10) présentant un réseau de transducteurs (8) et un réseau d'ADC (32), dans lequel la sonde US (10) est configurée pour insonifier une région d'imagerie (18), recevoir des signaux d'écho, numériser les signaux d'écho reçus et transférer les données RF numérisées (14) via une interface (36) comprenant un canal limité en bande passante vers une unité de traitement de données ( 12), dans lequel l'unité de traitement de données (12) est adaptée pour traiter et former en faisceau les données RF (14).

FIG. 1

FIG. 2

FIG. 3

FIG. 4

FIG. 6

24

8

34

32

42

10

14

50

36

44

12

40

26

FIG. 5

15

116

114

118

120

121

119

122

FIG. 7

FIG. 8

18a  18a  18a  18a  18a

154

16a

FIG. 9

18a   18a   18a   18a   18a
18b   18b  18b   18b
18b

154

16a

FIG. 10

T T T T
X X X X ⋯
1 2 3 1

154

18b

FIG. 11

FIG. 12

**REFERENCES CITED IN THE DESCRIPTION**

**Patent documents cited in the description**

- US 20160262720 A1

- US 5873830 A

**Non-patent literature cited in the description**

- **HENG-MING TAILS ; MEN LONG ; WEI HE ; HAO YARG.** AN EFFICIENT REGION OF INTEREST CODING FOR MEDICAL IMAGE COMPRESSION. *Proceedings of the Second Joint EMEISEIMES Conference Houston, TX, USA*

- Review on Region of Interest Coding Techniques for Medical Image Compression. *International Journal of Computer Applications,* January 2016, vol. 134 (10